# EUROPÄISCHE PATENTSCHRIFT

(11) **EP 1 148 617 B1**
(45) Veröffentlichungstag und Bekanntmachung des Hinweises auf die Patenterteilung: **22.08.2007**
(21) Anmeldenummer: 00119194.9
(22) Anmeldetag: 05.09.2000
(51) Int. Cl.: H02J 9/02, H02J 7/00, H02J 7/02, A61N 1/378

(54) **Implantierbare Energiespeicheranordnung für ein medizinisches Implantat sowie Betriebsverfahren dafür**
Implantable energy storage system for a medical implant and operating method therefor
Dispositif implantable d'accumulation d'énergie pour un implant medical et son procédé de fonctionnement

(30) Priorität: 03.04.2000 DE 10016520
(43) Veröffentlichungstag der Anmeldung: 24.10.2001
(73) Patentinhaber: Cochlear Limited, Lane Cove, NSW 2066 (AU)
(72) Erfinder: Boberschmidt, Werner, 85737 Ismaning (DE); Leysieffer, Hans Dr.-Ing., 82024 Taufkirchen (DE)
(74) Vertreter: Schwan, Gerhard

(56) Entgegenhaltungen:
- EP-A- 0 480 648
- DE-A- 19 838 137
- US-A- 5 279 292
- US-A- 5 330 515
- US-A- 5 528 201
- US-A- 5 576 612

## Beschreibung

Die vorliegende Erfindung betrifft eine implantierbare Energiespeicheranordnung für ein medizinisches Implantat sowie ein Verfahren zum Betreiben einer solchen Energiespeicheranordnung gemäß dem Oberbegriff von Anspruch 1 bzw. 20.

Solche gattungsgemäßen Energiespeicheranordnungen bzw. Verfahren sind beispielsweise aus DE 198 38 137 A1, US 5,411,537, US 5,702,431 sowie US 5,713,939 bekannt. Üblicherweise erfolgt das Nachladen von implantierbaren Energiespeicheranordnungen transkutan über eine induktive Strecke mittels eines externen Ladegeräts. Der Ladevorgang wird dabei üblicherweise dadurch gesteuert, dass der Ladestrom und die Spannung des Speichers von einer Steuereinheit gemessen und in entsprechende Steuerimpulse für einen Schalter im Ladestromkreis umgesetzt werden, wobei ein geeignetes Ladeprogramm verwendet wird.

Beim Betrieb des Energiespeichers können zwei unerwünschte Betriebszustände auftreten: Es kann einerseits eine Überladung der Batterie auftreten, wenn der Ladevorgang nicht rechtzeitig beendet wird, was zu Gasentwicklung mit nachfolgender Zerstörung des Speichers führen kann. Andererseits kann die Speicherspannung, wenn ein rechtzeitiges Aufladen des Speichers unterbleibt, auf Werte fallen, die unterhalb der minimalen Betriebsspannung liegen, welche für eine definierte Funktion oder gegebenenfalls zur eingeschränkten Funktion des von dem Energiespeicher zu versorgenden Implantats erforderlich ist. Unter Umständen kann in letzterem Fall die Speicherspannung so weit abfallen, dass auch eine ausreichende Spannungsversorgung der implantatseitigen Elektronik zum Steuern des Ladevorgangs nicht mehr gewährleistet ist. Häufig umfasst die Steuerelektronik ein Mikroprozessorsystem, bei welchem es im Unterspannungsbereich zur Ausführung von falschen logischen Operationen oder zum Verlust der Inhalts von flüchtigen Speichern kommen kann. Somit ist im Unterspannungsbereich bei solchen Systemen eine ordnungsgemäße Ladesteuerung nicht mehr gewährleistet, was dazu führen kann, dass beim Eintritt in den Unterspannungsbereich durch eine Mikroprozessor-Fehlfunktion der Ladepfad hochohmig geschaltet wird und ein Aufladen des Speichers auf diese Weise dauerhaft unterbunden wird.

Es ist Aufgabe der vorliegenden Erfindung, eine implantierbare Energiespeicheranordnung für ein medizinisches Implantat sowie ein Verfahren zum Betrieb derselben zu schaffen, wobei ein sicheres Wiederaufladen des Energiespeichers auch dann möglich ist, wenn der Speicher vollständig oder zumindest so weit entladen wurde, dass die Speicherspannung unter den Normalspannungsbereich für die Steuereinheit abgefallen war.

Diese Aufgabe wird erfindungsgemäß gelöst durch eine implantierbare Energiespeicheranordnung gemäß Anspruch 1 sowie ein entsprechendes Betriebsverfahren gemäß Anspruch 20. Bei dieser erfindungsgemäßen Lösung ist vorteilhaft, dass auch bei weit fortgeschrittener und sogar vollständiger Entladung des Speichers, d.h. bei sehr niedrigen Speicherspannungen, die Wiederaufladbarkeit des Speichers jederzeit sichergestellt ist, indem eine Blockade des Ladepfads wegen einer unterspannungsbedingten Fehlfunktion der Steuereinheit verhindert wird mittels der Überbrückungsmöglichkeit des Stellglieds im Ladepfad durch externe Betätigung der Überbrückungseinheit.

Bevorzugte Ausgestaltungen der Erfindung ergeben sich aus den Unteransprüchen.

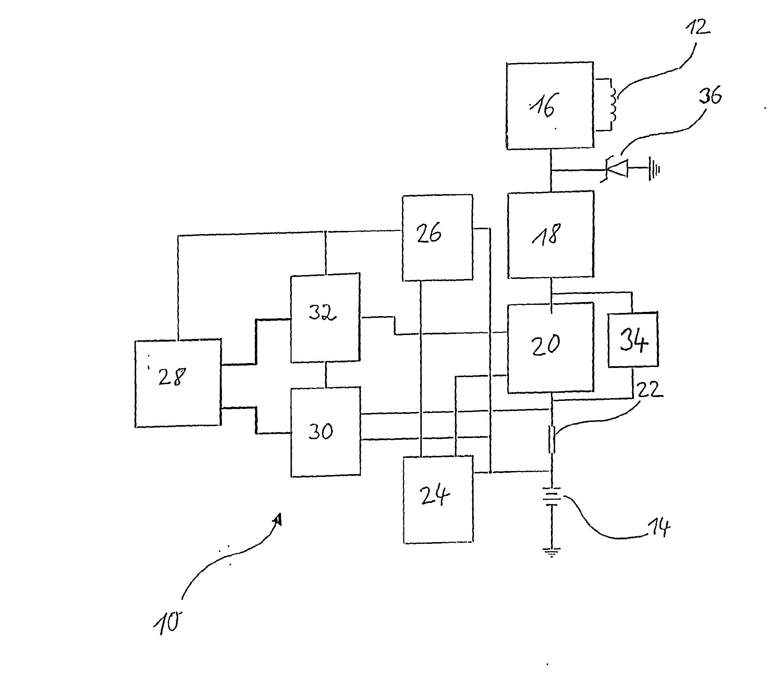

Im folgenden ist eine Ausführungsform der Erfindung anhand der beigefügten Zeichnung beispielhaft näher erläutert, welche ein Blockschaltbild einer erfindungsgemäßen implantierbaren Speicheranordnung für ein medizinisches Implantat zeigt.

Die einzige Figur zeigt schematisch die Verschaltung der wesentlichen Elemente einer implantierbaren Speicheranordnung 10, die als Stromversorgung für ein medizinisches Implantat dient, bei welchem es sich beispielsweise um ein voll implantierbares Hörsystem für die direkte mechanische Stimulation des Mittel- oder Innenohres oder die elektrische Stimulation des Innenohres handeln kann. Eine Ladespule 12 dient dazu, von einem nicht dargestellten externen Ladegerät induktiv transkutan übertragene elektrische Energie aufzunehmen und in den Ladepfad eines wiederaufladbaren Energiespeichers 14, bei dem es sich beispielsweise um eine NiMH-Batterie handeln kann, einzuspeisen. Die in der Ladespule 2 von dem externen Ladegerät induzierte Spannung wird in einer Einheit 16 gleichgerichtet und aufbereitet. Eine Zenerdiode 36 ist vorgesehen, um auf Ladespannungspotential liegende elektronische Bauelemente vor einer zu hohen Ladespannung zu schützen.

Der Ladestrom fließt durch einen im Normalbetrieb geschlossenen mechanischen Schalter 18, ein Regel- und Schaltglied 20, bei dem es sich vorzugsweise im wesentlichen um einem FET handelt und das als steuerbarer Widerstand im Ladestrompfad wirkt, und einen Shuntwiderstand 22 zur Batterie 14. Eine Überwachungseinheit 24 ist direkt mit der Batterie 14 verbunden und dient zur Erfassung und Überwachung der Batteriespannung. Ausgangsseitig ist die Überwachungseinheit 24 einerseits mit dem Regel- und Schaltglied 20 und andererseits mit einem Schalter 26 verbunden, über welchen ein Mikroprozessorsystem 28 mit der Batterie 14 als Spannungsversorgung verbunden ist. Das Mikroprozessorsystem 28 wird vorzugsweise von einem (nicht dargestellten) Pierce-Oszillator getaktet. Ein A/D-Wandler 30 dient dazu, die Spannung der Batterie 14 sowie den Spannungsabfall an dem Shuntwiderstand 22 zu messen, wodurch die beiden wichtigsten Parameter beim Aufladen der Batterie 14, nämlich die Batteriespannung und der Ladestrom, gemessen werden können. Diese Werte werden als Ausgabe des A/D-Wandlers 30 dem Mikroprozessorsystem 28 als Eingangssignal zugeführt, welcher in Abhängigkeit von der erfassten Batteriespannung und dem erfassten Ladestrom über einen D/A-Wandler 32 das Regel/Schaltglied 20 und damit den Ladestrom bzw. die Ladespannung entsprechend einer vorgegebenen Ladestrategie steuert. Insbesondere ist dabei in bekannter Weise eine Überwachungsfunktion implementiert, die dafür sorgt, dass nach Erreichen eines vorgegebenen Ladeendkriteriums der Aufladevorgang beendet wird, indem das Regel/Schaltglied 20 hochohmig geschaltet wird.

Um eine übermäßige Entladung der Batterie 14 im Betrieb zu verhindern, ist die Energiespeicheranordnung 10 in an sich bekannter Weise mit einer Funktion versehen, welche den Implantatträger rechtzeitig vor einer Entladung der Batterie 14 warnt, um ihn zu der Vornahme eines Aufladevorgangs zu animieren. Falls eine Wiederaufladung nicht erfolgt, weil beispielsweise der Implantatträger verhindert ist, kann die Batteriespannung unter die zulässige Untergrenze absinken. Sobald die Überwachungseinheit 24 feststellt, dass die von ihr erfasste Batteriespannung einen vorgegebenen unteren Schwellwert unterschritten hat, schaltet die Überwachungseinheit 24 einerseits durch Öffnen des Schalters 26 den Mikroprozessor 28 ab, indem dessen Spannungsversorgung unterbrochen wird. Dies stellt sicher, dass der Mikroprozessor 28 nicht bei Versorgungsspannungen betrieben wird, die so niedrig sind, dass der Mikroprozessor 28 falsche logische Operationen ausführen oder den Inhalt von flüchtigen Speichern verlieren kann. Dies wird durch entsprechende Wahl der unteren Spannungsgrenze sichergestellt. Andererseits setzt die Überwachungseinheit 24 bei Unterschreiten der Spannungsuntergrenze das Regel/Schaltglied 20 in einen leitenden Zustand, um sicherzustellen, dass eine Aufladung der Batterie 14 auch im Unterspannungsbereich jederzeit möglich ist und keine Blockade des Ladepfads durch eine Fehlfunktion des Mikroprozessors 28 möglich ist. Als zusätzlicher Effekt durch das Abschalten des Mikroprozessors 28 ergibt sich, dass die Stromaufnahme der Elektronik auf die Stromaufnahme der Überwachungseinheit 24 gesenkt wird, die in der Praxis nur einige 100 nA beträgt. Dies hat den positiven Effekt, dass die Batterie 14 weniger belastet wird und dadurch der Zeitraum bis zur vollständigen Entladung der Batterie 14 verlängert wird, was das Risiko einer vollständigen Entladung verringert.

Bei einem im Unterspannungsbereich vorgenommenen Aufladevorgang steigt die Spannung der Batterie 14 allmählich an. Nachdem die Batteriespannung den vorgegebenen unteren Spannungsgrenzwert der Überwachungseinheit 24 um den Wert der Hysterese überschritten hat, schaltet die Überwachungseinheit 24 durch Schließen des Schalters 26 das Mikroprozessorsystem 28 wieder ein, wodurch dieses beginnt, den Aufladevorgang in der oben beschriebenen Weise zu steuern und zu überwachen. Dadurch wird die Steuerung des Regel/Schaltglieds 20 von der Überwachungseinheit 24 an das Mikroprozessorsystem 28 übergeben. Durch das sprunghafte Einschalten der Versorgungsspannung durch das Schließen des Schalters 26 wird ein steiler Spannungsanstieg realisiert, welcher das Anschwingen des Pierce-Oszillators des Mikroprozessorsystems 28 in sicherer Weise gewährleistet.

Ferner ist die Überwachungseinheit 24 so ausgebildet, dass sie, wenn die von ihr erfasste Batteriespannung einen vorbestimmten Maximalwert überschreitet, das Regel/Schaltglied 20 nichtleitend schaltet, um eine Überladung der Batterie 14 selbst dann zu verhindern, wenn das Mikroprozessorsystem 28 aufgrund eines Fehlers den Ladevorgang nach Erreichen des vorgegebenen Ladeendkriteriums nicht beendet.

Der mechanische Schalter 18 in an sich bekannter Weise so ausgebildet, dass er auf eine mechanische Ausdehnung der Batterie 14, wie sie bei der mit einer Überladung einhergehenden übermäßigen Gasentwicklung auftritt, anspricht und den Ladepfad unterbricht oder den Empfang von Ladeenergie verhindert, um ein weiteres Aufladen der Batterie 14 zu verhindern.

Auf diese Weise sind bei der beschriebenen Speicheranordnung 10 drei unabhängige Überwachungskreise realisiert, welche die Aufladung der Batterie rechtzeitig beenden, um Schäden zu vermeiden. Dabei handelt es sich primär um das Mikroprozessorsystem 28, welches den Ladevorgang bei Erreichen eines vorgegebenen Ladeendkriteriums beendet. Unabhängig davon beendet die Überwachungseinheit 24 den Ladevorgang, wenn die von ihr unabhängig von dem Mikroprozessorsystem 28 erfasste Batteriespannung einen vorgegebenen Maximalwert überschreitet. Schließlich beendet der mechanische Schalter 18 bei Versagen der ersten beiden Überwachungskreise elektronikunabhängig den Ladevorgang so rechtzeitig, dass eine Beschädigung der Batterie 14 und eine Gefährdung des Implantatträgers zuverlässig verhindert wird.

Die Überwachungseinheit 24 sorgt ferner dafür, dass unabhängig von dem Mikroprozessorsystem 28 eine drohende übermäßige Entladung der Batterie 14 erkannt wird und möglicherweise daraus resultierende Fehlfunktionen des Mikroprozessorsystems 28 durch Abschalten desselben verhindert werden. Ferner übernimmt die Überwachungseinheit 24 in diesem Fall die Steuerung des Ladepfads, wobei jederzeit unabhängig von dem Mikroprozessorsystem 28 im Unterspannungsbereich sichergestellt wird, dass der Ladepfad leitend ist, so dass jederzeit eine Wiederaufladung der Batterie 14 möglich ist. Durch das schlagartige Wiedereinschalten der Versorgungsspannung des Mikroprozessorsystems 28 durch Schließen des Schalters 26 durch die Überwachungseinheit 24 können ferner Anlaufprobleme des Pierce-Oszillators des Mikroprozessorsystems 28 verhindert werden.

Parallel zu dem Regel/Schaltglied 20 ist eine Überbrückungseinrichtung 34 geschaltet, die extern, d.h. von außerhalb des Körpers betätigbar ist, um das Regel/Schaltglied 20 zu überbrücken bzw. kurzzuschließen. Die Überbrückungseinrichtung 34 ist für den Fall vorgesehen, wenn der Speicher 14 soweit entladen ist, dass die Speicherspannung so weit abgefallen ist, dass sie für eine ordnungsgemäße Funktion der Überwachungseinheit 24 nicht mehr ausreichend ist. Dabei kann der Fall eintreten, dass sich das Regel/Schaltglied 20 in einen Zustand mit so hohem Widerstand kommt, der ein Aufladen des Speichers 14 über den normalen Ladepfad nicht ermöglicht. In diesem Fall kann durch Betätigung der Überbrückungseinrichtung 34 von außen eingegriffen werden, um das Regel/Schaltglied 20 zu überbrücken und so ein sicheres Aufladen des Speichers 20 auch bei extremer Unterspannung oder vollständiger Entladung zu ermöglichen.

Die Überbrückungseinrichtung 34 kann beispielsweise von einem mittels eines externen Magneten schließbaren mechanischen Schalter gebildet werden (Reed-Schalter), wobei der Magnet vorteilhafterweise in das externe Ladegerät integriert sein kann. In diesem Fall wird, wenn das Ladegerät an die Haut des Implantatträgers gehalten wird, der Schalter 34 geschlossen und ein Aufladen ist über den über den Schalter 34 führenden Strompfad möglich. Sobald jedoch die Speicherspannung für einen sicheren Betrieb der Überwachungseinheit 24 wieder ausreicht, sollte der Schalter 34 durch Entfernen des Magneten wieder geöffnet werden, um die oben beschriebene normale Ladefunktion wieder zu aktivieren und insbesondere ein Überladen zu verhindern.

In alternativer Ausgestaltung kann die Überbrückungseinrichtung 34 auch von einer in Sperrrichtung gepolten Diode, beispielsweise einer Zenerdiode, gebildet werden. Die Sperrspannung der Diode ist dabei so bemessen, dass sie oberhalb der im normalen Lademodus auftretenden Ladespannungen liegt und so den normalen Ladevorgang nicht beeinflusst, jedoch unterhalb der Sperrspannung der Schutzdiode 36 liegt. Im Notfall, d.h. wenn die Speicherspannung nicht mehr zum Betrieb der Überwachungseinheit 24 ausreicht, wird dann zum Beginn des Wiederaufladens ein spezielles externes Notladegerät verwendet, welches sich von dem für den normalen Ladevorgang verwendeten externen Ladegerät im wesentlichen dadurch unterscheidet, dass es dafür sorgt. dass eine Ladespannung erzeugt wird, die wesentliche höher als beim normalen Ladevorgang ist und oberhalb der Sperrspannung der Überbrückungsdiode 34, jedoch unterhalb der Sperrspannung der Schutzdiode 36 liegt. Auf diese Weise wird mit dem Notladegerät eine Überbrückung des Regel/Schaltglieds 20 erreicht. Statt der Verwendung eines speziellen Notladegeräts kann auch des normale Ladegerät mit einem umschaltbaren Notlademodus versehen sein. Jedoch sollte bei Erreichen einer Speicherspannung, die für einen sicheren Betrieb der Überwachungseinheit 24 wieder ausreicht, der Notlademodus beendet werden, um die Überbrückungsdiode 34 wieder sperrend zu machen und somit die normale Ladefunktion wieder zu aktivieren und insbesondere ein Überladen zu verhindern.

Mittels der Überbrückungseinheit 34 wird somit sichergestellt, dass selbst bei vollständig entladenem Speicher 14 durch externe Betätigung ein Wiederaufladen möglich ist.

## Patentansprüche

1. Implantierbare Energiespeicheranordnung für ein medizinisches Implantat, mit einem wiederaufladbaren Speicher (14) für elektrische Energie sowie einer Einheit (20, 22, 26, 28, 30, 32) zum Steuern des Ladevorgangs über ein Stellglied (20) im Ladepfad, **dadurch gekennzeichnet, dass** eine Einrichtung (34) vorgesehen ist, welche extern betätigbar ist, um das Stellglied (20) zu überbrücken.

2. Speicheranordnung nach Anspruch 1, **dadurch gekennzeichnet, dass** es sich bei der Überbrückungseinrichtung (34) um einen magnetisch betätigbaren Schalter handelt.

3. Speicheranordnung nach Anspruch 1, **dadurch gekennzeichnet, dass** es sich bei der Überbrückungseinrichtung (34) um eine in Sperrrichtung gepolte Diode handelt, deren Sperrspannung größer als die beim normalen Ladevorgang auftretenden Ladespannungen ist.

4. Speicheranordnung nach Anspruch 3, **dadurch gekennzeichnet, dass** eine Schutzdiode (36) für die Steuereinheit (20, 22, 26, 28, 30, 32) vorgesehen ist, wobei die Sperrspannung der Schutzdiode größer als diejenige der Überbrückungsdiode (34) ist.

5. Speicheranordnung nach einem der vorhergehenden Ansprüche, **dadurch gekennzeichnet, dass** das Stellglied (20) von einem steuerbaren Widerstand gebildet wird.

6. Speicheranordnung nach einem der vorhergehenden Ansprüche, **dadurch gekennzeichnet, dass** eine von der Steuereinheit (20, 22, 26, 28, 30, 32) unabhängige Überwachungseinheit (24) vorgesehen ist, welche die Speicherspannung unabhängig von der Steuereinheit erfasst und so ausgebildet ist, dass sie, wenn die von ihr erfasste Speicherspannung außerhalb eines vorgegebenen Bereichs liegt, die Steuerung des Ladepfads übernimmt.

7. Speicheranordnung nach Anspruch 6, **dadurch gekennzeichnet, dass** die Überwachungseinheit (24) so ausgebildet ist, dass sie, wenn die von ihr erfasste Speicherspannung einen vorbestimmten ersten unteren Schwellwert unterschreitet, dafür sorgt, dass der Ladepfad so geschaltet ist, dass der Speicher (14) mit einem Ladestrom beaufschlagbar ist.

8. Speicheranordnung nach Anspruch 7, **dadurch gekennzeichnet, dass** die Überwachungseinheit (24) so ausgebildet ist, dass das Stellglied (20) von der Überwachungseinheit in den leitenden Zustand gebracht wird, wenn die von der Überwachungseinheit erfasste Speicherspannung den vorbestimmten ersten unteren Schwellwert unterschreitet.

9. Speicheranordnung nach Anspruch 8, **dadurch gekennzeichnet, dass** die Steuereinheit (20, 22, 26, 28, 30, 32) ein Mikroprozessorsystem (28) enthält, das über einen ersten Schalter (26) von dem Speicher (14) mit Spannung versorgt wird, wobei die Überwachungseinheit (24) so ausgebildet ist, dass sie, wenn die erfasste Speicherspannung einen vorbestimmten zweiten unteren Schwellwert unterschreitet, das Mikroprozessorsystem durch Öffnen des zweiten Schalters von der Spannungsversorgung durch den Speicher abtrennt.

10. Speicheranordnung nach Anspruch 9, **dadurch gekennzeichnet, dass** der erste und der zweite untere Schwellwert identisch sind.

11. Speicheranordnung nach Anspruch 9 oder 10, **dadurch gekennzeichnet, dass** die Überwachungseinheit (24) so ausgebildet ist, dass sie, wenn die erfasste Speicherspannung einen vorbestimmten ersten oberen Schwellwert überschreitet, das Mikroprozessorsystem (28) durch Schließen des ersten Schalters (26) an die Spannungsversorgung durch den Speicher (14) anschließt und die Steuerung des steuerbaren Widerstands (20) an das Mikroprozessorsystem übergibt.

12. Speicheranordnung nach einem der Ansprüche 9 bis 11, **dadurch gekennzeichnet, dass** das Mikroprozessorsystem (28) von einem Pierce-Oszillator getaktet wird.

13. Speicheranördnung nach Anspruch 8 bis 12, **dadurch gekennzeichnet, dass** die Steuereinheit (20, 22, 26, 28, 30, 32) eine Einrichtung (30) zum Erfassen der Speicherspannung und des Stroms im Ladestromkreis umfasst und das Stellglied (20) gemäß einem Ladeprogramm in Abhängigkeit von den so erfassten Werten steuert.

14. Speicheranordnung nach einem der Ansprüche 6 bis 13, **dadurch gekennzeichnet, dass** die Überwachungseinheit (24) so ausgebildet ist, dass sie, wenn die Speicherspannung einen vorbestimmten Maximalwert überschreitet, den Ladepfad so schaltet, dass eine weitere Beaufschlagung des Speichers (14) mit Ladestrom verhindert wird.

15. Speicheranordnung nach Anspruch 14, **dadurch gekennzeichnet, dass** die Überwachungseinheit (24) so ausgebildet ist, dass sie, wenn die Speicherspannung den vorbestimmten Maximalwert überschreitet, den steuerbaren Widerstand (20) nichtleitend schaltet.

16. Speicheranordnung nach einem der vorhergehenden Ansprüche, **dadurch gekennzeichnet, dass** ein zweiter Schalter (18) vorgesehen ist, der auf mechanische Veränderungen des Speichers (14) durch Überladung anspricht und dabei den Speicher von der Ladestromquelle abkoppelt oder den Empfang von Ladeenergie verhindert.

17. Speicheranordnung nach Anspruch 16, **dadurch gekennzeichnet, dass** der zweite Schalter (18) auf eine Ausdehnung des Speichers (14) anspricht und dabei den Ladestrompfad unterbricht oder den Empfang von Ladeenergie verhindert.

18. Speicheranordnung nach einem der vorhergehenden Ansprüche, **dadurch gekennzeichnet, dass** eine Einrichtung (12, 16) vorgesehen ist, um Ladeenergie, die induktiv von einer externen Quelle transkutan übertragen wird, aufzunehmen und für eine Einspeisung in den Ladestrompfad aufzubereiten.

19. Speicheranordnung nach einem der vorhergehenden Ansprüche, **dadurch gekennzeichnet, dass** es sich bei dem medizinischen Implantat um ein vollimplantierbares Hörsystem handelt.

20. Verfahren zum Betreiben einer implantierbaren Energiespeicheranordnung für ein medizinisches Implantat, mit einem wiederaufladbaren Speicher (14) für elektrische Energie, wobei im Normalbetrieb der Ladevorgang mittels einer Steuereinheit (20, 22, 26, 28, 30, 32) über ein Stellglied (20) im Ladepfad gesteuert wird, **dadurch gekennzeichnet, dass**, wenn ein Aufladen des Speichers (14) über das Stellglied (20) aufgrund zu geringer Speicherspannung nicht möglich ist, eine in der implantierbaren Energiespeicheranordnung vorgesehene Überbrückungseinrichtung (34) von extern betätigt wird, um das Stellglied (20) zu überbrücken.

## Claims

1. Implantable energy storage arrangement for a medical implant, with a rechargeable storage (14) for electrical energy and a unit (20, 22, 26, 28, 30, 32) for controlling the charging process via an actuator (20) in the charging path, **characterized in that** there are provided means (34) which can be externally activated to bypass the actuator (20).

2. Storage arrangement as claimed in claim 1, **characterized in that** the bypass means (34) is a magnetically actuatable switch.

3. Storage arrangement as claimed in claim 1, **characterized in that** the bypass means (34) is a diode which is poled in the reverse direction and which has a reverse voltage which is greater than the charging voltages which occur in a normal charging process.

4. Storage arrangement as claimed in claim 3, **characterized in that** there is a protective diode (36) for the control unit (20, 22, 26, 28, 30, 32), the reverse voltage of the protective diode being greater than that of the bypass diode (34).

5. Storage arrangement as claimed in any one of the preceding claims, **characterized in that** the actuator (20) comprises a controllable resistor.

6. Storage arrangement as claimed in any one of the preceding claims, **characterized in that** there is provided a monitoring unit (24) which is independent of the control unit (20, 22, 26, 28, 30, 32) and which senses the storage voltage independently of the control unit and is designed such that it assumes control of the charging path when the storage voltage sensed by it lies outside a predetermined range.

7. Storage arrangement as claimed in claim 6, **characterized in that** the monitoring unit (24) is made such that when the storage voltage sensed by it falls below a predetermined first lower threshold it provides for switching the charging path such that a charging current can be supplied to the storage (14).

8. Storage arrangement as claimed in claim 7, **characterized in that** the monitoring unit (24) is made such that the actuator (20) is brought into the conductive state by the monitoring unit when the storage voltage sensed by the monitoring unit falls below the predetermined first lower threshold.

9. Storage arrangement as claimed in claim 8, **characterized in that** the control unit (20, 22, 26, 28, 30, 32) contains a microprocessor system (28) which is supplied with voltage via a first switch (26) by the storage (14), the monitoring unit (24) being made such that when the sensed storage voltage falls below a predetermined second lower threshold it cuts off the microprocessor system from the voltage supply by the storage by opening the second switch.

10. Storage arrangement as claimed in claim 9, **characterized in that** the first and the second lower thresholds are identical.

11. Storage arrangement as claimed in claim 9 or 10, **characterized in that** the monitoring unit (24) is made such that when the sensed storage voltage exceeds a predetermined first upper threshold it connects the microprocessor system (28) to the voltage supply by the storage (14) by closing the first switch (26) and transfers control of the controllable resistor (20) to the microprocessor system.

12. Storage arrangement as claimed in any one of claims 9 to 11, **characterized in that** the microprocessor system (28) is clocked by a Pierce oscillator.

13. Storage arrangement as claimed in any one of claims 8 to 12, **characterized in that** the control unit (20, 22, 26, 28, 30, 32) comprises a means (30) for sensing the storage voltage and the current in the charging circuit and controls the actuator (20) according to a charging program as a function of the values sensed in this way.

14. Storage arrangement as claimed in any one of claims 6 to 13, **characterized in that** the monitoring unit (24) is made such that when the storage voltage exceeds a predetermined maximum value it switches the charging path such that charging current is prevented from flowing to the storage (14).

15. Storage arrangement as claimed in claim 14, **characterized in that** the monitoring unit (24) is made such that when the storage voltage exceeds a predetermined maximum value it switches the controllable resistor (20) non-conducting.

16. Storage arrangement as claimed in any one of the preceding claims, **characterized in that** there is a second switch (18) which responds to mechanical changes of the storage (14) by overcharging and in doing so decouples the storage from the charging current source or prevents reception of charging energy.

17. Storage arrangement as claimed in claim 16, **characterized in that** the second switch (18) responds to an expansion of the storage (14) and in doing so interrupts the charging current path or prevents reception of charging energy.

18. Storage arrangement as claimed in any one of the preceding claims, **characterized in that** there is a means (12, 16) for picking up charging energy which is inductively transmitted transcutaneously from an external source and for preparing the charging energy for feed to the charging current path.

19. Storage arrangement as claimed in any one of the preceding claims, **characterized in that** the medical implant is a fully implantable hearing system.

20. Process for operating an implantable energy storage arrangement for a medical implant, with a rechargeable storage (14) for electrical energy, in normal operation the charging operation being controlled by means of a control unit (20, 22, 26, 28, 30, 32) via an actuator (20) in the charging path, **characterized in that** when it is not possible to charge the storage (14) via the actuator (20) due to overly low storage voltage, a bypass means (34) provided in the implantable energy storage arrangement is activated from the outside to bypass the actuator (20).

## Revendications

1. Dispositif accumulateur d'énergie implantable pour un implant médical, comprenant un accumulateur (14) rechargeable pour de l'énergie électrique et une unité (20, 22, 26, 28, 30, 32) pour la commande de l'opération de chargement au moyen d'un actionneur (20) dans le chemin de chargement, **caractérisé en ce qu'**il est prévu un appareil (34) qui peut être actionné par l'extérieur afin de shunter l'actionneur (20).

2. Dispositif accumulateur selon la revendication 1, **caractérisé en ce que**, en ce qui concerne le dispositif de l'appareil de shuntage (34), il s'agit d'un interrupteur pouvant être actionné de façon magnétique.

3. Dispositif accumulateur selon la revendication 1, **caractérisé en ce que**, en ce qui concerne le dispositif de l'appareil de shuntage (34), il s'agit d'une diode polarisée dans le sens de blocage, dont la tension de blocage est supérieure aux tensions de chargement qui apparaissent lors de l'opération de chargement normale.

4. Dispositif accumulateur selon la revendication 3, **caractérisé en ce qu'**une diode de protection (36) est prévue pour l'unité de commande (20, 22, 26, 28, 30, 32), la tension de blocage de la diode de protection étant supérieure à celle de la diode de shuntage (34).

5. Dispositif accumulateur selon l'une quelconque des revendications précédentes, **caractérisé en ce que** l'actionneur (20) est formé par une résistance contrôlable.

6. Dispositif accumulateur selon l'une quelconque des revendications précédentes, **caractérisé en ce qu'**il est prévu une unité de surveillance (24) indépendante de l'unité de commande (20, 22, 26, 28, 30, 32) qui saisit la tension de l'accumulateur indépendamment de l'unité de commande et est conçue de telle sorte qu'elle prend en charge la commande du chemin de chargement lorsque la tension de stockage qu'elle saisit se situe en dehors d'une plage prédéfinie.

7. Dispositif accumulateur selon la revendication 6, **caractérisé en ce que** l'unité de surveillance (24) est conçue de telle sorte que, lorsque la tension de stockage saisie par elle est inférieure à une première valeur seuil prédéfinie, elle garantit que le chemin de chargement est commuté de telle sorte que l'accumulateur (14) peut être sollicité avec un courant de chargement.

8. Dispositif accumulateur selon la revendication 7, **caractérisé en ce que** l'unité de surveillance (24) est réalisée de telle sorte que l'actionneur (20) est amené de l'unité de surveillance dans l'état conducteur lorsque la tension de stockage saisie par l'unité de surveillance est inférieure à la première valeur seuil prédéfinie.

9. Dispositif accumulateur selon la revendication 8, **caractérisé en ce que** l'unité de commande (20, 22, 26, 28, 30, 32) contient un système de microprocesseur (28) qui est alimenté en tension par l'accumulateur (14) par le biais d'un premier interrupteur (26), l'unité de surveillance (24) étant réalisée de telle sorte que, lorsque la tension de stockage saisie est inférieure à une seconde valeur seuil prédéfinie, elle sépare le système du microprocesseur par l'ouverture du second interrupteur de la tension d'alimentation par l'accumulateur.

10. Dispositif accumulateur selon la revendication 9, **caractérisé en ce que** les première et seconde valeurs seuils inférieures sont identiques.

11. Dispositif accumulateur selon la revendication 9 ou 10, **caractérisé en ce que** l'unité de surveillance (24) est réalisée de telle sorte que, lorsque la tension de stockage saisie dépasse une première valeur seuil supérieure prédéfinie, elle raccorde le système de microprocesseur (28) par la fermeture du premier interrupteur (26) à la tension d'alimentation par l'accumulateur (14) et transmet la commande de la résistance (20) contrôlable au système de microprocesseur.

12. Dispositif accumulateur selon l'une quelconque des revendications 9 à 11, **caractérisé en ce que** le système de microprocesseur (28) est cadencé par un oscillateur de Pierce.

13. Dispositif accumulateur selon les revendications 8 à 12, **caractérisé en ce que** l'unité de commande (20, 22, 26, 28, 30, 32) comprend un appareil (30) pour la saisie de la tension de stockage et du courant dans le circuit de chargement et commande l'actionneur (20) selon un programme de chargement en fonction des valeurs ainsi saisies.

14. Dispositif accumulateur selon l'une quelconque des revendications 6 à 13, **caractérisé en ce que** l'unité de surveillance (24) est réalisée de telle sorte que, lorsque la tension d'accumulateur dépasse une valeur maximale prédéfinie, elle commute le chemin de chargement de sorte qu'une nouvelle alimentation de l'accumulateur (14) avec du courant de chargement est empêchée.

15. Dispositif accumulateur selon la revendication 14, **caractérisé en ce que** l'unité de surveillance (24) est conçue de telle sorte que, lorsque la tension d'accumulateur dépasse la valeur maximale prédéfinie, elle commute la résistance (20) contrôlable de façon non conductrice.

16. Dispositif accumulateur selon l'une quelconque des revendications précédentes, **caractérisé en ce qu'**il est prévu un second interrupteur (18) qui réagit à des variations mécaniques de l'accumulateur (14) par surcharge et dissocie ainsi l'accumulateur de la source du courant de chargement ou empêche la réception d'énergie de chargement.

17. Dispositif accumulateur selon la revendication 16, **caractérisé en ce que** le second interrupteur (18) réagit à une extension de l'accumulateur (14) et interrompt ainsi le chemin du courant de charge ou empêche la réception d'énergie de chargement.

18. Dispositif accumulateur selon l'une quelconque des revendications précédentes, **caractérisé en ce qu'**il est prévu un appareil (12, 16) pour réceptionner de l'énergie de chargement qui est transmise de façon inductive par une source externe sous une forme transcutanée et la traiter pour une injection dans le chemin de courant de chargement.

19. Dispositif accumulateur selon l'une quelconque des revendications précédentes, **caractérisé en ce que**, en ce qui concerne l'implant médical, il s'agit d'un système auditif entièrement implantable.

20. Procédé pour exploiter un dispositif accumulateur d'énergie implantable destiné à un implant médical, comprenant un accumulateur (14) rechargeable pour de l'énergie électrique, l'opération de chargement étant commandée en fonctionnement normal au moyen d'une unité de commande (20, 22, 26, 28, 30, 32) par le biais d'un actionneur (20) dans le chemin de chargement, **caractérisé en que**, lorsqu'un rechargement de l'accumulateur (14) par l'actionneur (20) est impossible en raison de la tension d'accumulateur trop faible, un dispositif de shuntage (34) prévu dans le dispositif accumulateur d'énergie implantable est actionné par l'extérieur afin de shunter l'actionneur (20).
